**Europäisches Patentamt**

**European Patent Office**

**Office européen des brevets**

(19)

(11) Veröffentlichungsnummer: **0 273 256**

**A1**

(12) # EUROPÄISCHE PATENTANMELDUNG

(21) Anmeldenummer: 87118235.8

(22) Anmeldetag: 09.12.87

(51) Int. Cl.⁴: **A61B 17/22** , A61B 6/10

(30) Priorität: 22.12.86 DE 3643976

(43) Veröffentlichungstag der Anmeldung:
06.07.88 Patentblatt 88/27

(84) Benannte Vertragsstaaten:
DE FR GB NL

(71) Anmelder: **Siemens Aktiengesellschaft Berlin und München**
**Wittelsbacherplatz 2**
**D-8000 München 2(DE)**

(72) Erfinder: **Grasser, Franz, Dipl.-Ing,**
**Neuwiesenstrasse 27**
**D-8557 Eggolsheim(DE)**

(54) Vorrichtung zum berührungslosen Zertrümmern von Konkrementen.

(57) Die Erfindung betrifft eine Vorrichtung zum berührungslosen Zertrümmern von Konkrementen (29) im Körper eines Patienten (4) mit einem Stoßwellengenerator (20) zur Erzeugung von fokussierten Stoßwellen innerhalb eines in einem Abstand vor dem Stoßwellengenerator (20) liegenden Wirkungsbereiches, wobei der Stoßwellengenerator (20) einen festen Teil (33, 36) und einen mit Koppelflüssigkeit (32) gefüllten Teil (35) aufweist, mit einer Bewegungsvorrichtung zum Ankoppeln des Stoßwellengenerators (20) an einem auf einer Patientenlagerung befindlichen Patienten (4), durch die der Stoßwellengenerator (20) und die Patientenlagerung relativ zueinander bewegt werden, und mit einer Ortungsvorrichtung zur Darstellung der Konkremente (29) mit einer Markierungsvorrichtung für die Konkremente (29). Der Stoßwellengenerator (20) weist einen Detektor (26, 41) auf, der eine Berührung des Patienten (4) durch den festen Teil (33, 36) des Stoßwellengenerators (20) erkennt und derart mit der Bewegungsvorrichtung verbunden ist, daß der Ankoppelvorgang gestoppt werden kann.

FIG 4

EP 0 273 256 A1

## Vorrichtung zum berührungslosen Zertrümmern von Konkrementen

Die Erfindung betrifft eine Vorrichtung zum berührungslosen Zertrümmern von Konkrementen im Körper eines Patienten mit einem Stoßwellengenerator zur Erzeugung von fokussierten Stoßwellen innerhalb eines in einem Abstand vor dem Stoßwellengenerator liegenden Wirkungsbereiches, wobei der Stoßwellengenerator einen festen Teil und einen mit Koppelflüssigkeit gefüllten Teil aufweist, mit einer Bewegungsvorrichtung zum Ankoppeln des Stoßwellengenerators an einem auf einer Patientenlagerung befindlichen Patienten, durch die der Stoßwellengenerator und die Patientenlagerung relativ zueinander bewegt werden, und mit einer Ortungsvorrichtung zur Darstellung der Konkremente mit einer Markierungsvorrichtung für die Konkremente. Vorrichtungen dieser Art werden in der Medizin beispielsweise zum Zerstören von Steinen in der Niere des Menschen eingesetzt. Sie sind besonders vorteilhaft, da sie jeglichen Eingriff in den Körper vermeiden.

In der DE-OS 33 28 051 ist eine derartige Vorrichtung beschrieben, die einen Stoßwellengenerator aufweist, der aus einem rohrförmigen Mantel, einer Flachspule und einer durch eine Isolierfolie getrennte Kupfermembran besteht. In dem Stoßwellengenerator ist eine akustische Sammellinse angeordnet, die die von der Membran erzeugten ebenen Stoßwellen in einem Brennpunkt fokussiert. Zur Ankopplung des Stoßwellengenerators an den Patienten ist die der Membran gegenüberliegende Öffnung des Stoßwellenrohres mit einem Sack abgeschlossen, der wie der gesamte Stoßwellengenerator mit einem Koppelmedium gefüllt ist. Zur Ankopplung wird der Stoßwellengenerator so lange auf den Patienten zu gefahren, bis daß das zu zerstörende Konkrement sich im Brennpunkt der Linsenanordnung befindet. Hierbei legt sich der mit der Koppelflüssigkeit gefüllte Sack an die Oberfläche des Patienten an, so daß gewährleistet ist, daß die Stoßwellen immer innerhalb der Flüssigkeit verlaufen.

Bei korpulenten Patienten tritt aber das Problem auf, daß das zu zerstörende Konkrement so weit innerhalb des Körpers des Patienten liegt, daß der Fokusabstand der Linsenanordnung nicht mehr ausreicht. Dadurch berührt der Stoßwellengenerator bzw. die Linsenanordnung den Patienten, so daß bei Fortsetzung des Ankoppelvorganges der Patient zur Seite geschoben wird. Dadurch wird das Konkrement aus den Fokus bewegt, so daß eine Behandlung nicht mehr erfolgen kann.

Die Erfindung geht von der Aufgabe aus, eine Vorrichtung der eingangs genannten Art derart auszubilden, daß der Einsatzbereich des Stoßwellengenerators erweitert wird, so daß auch korpulente Patienten behandelt werden können.

Die Aufgabe wird erfindungsgemäß dadurch gelöst, daß der Stoßwellengenerator einen Detektor aufweist, der eine Berührung des Patienten durch den festen Teil des Stoßwellengenerators erkennt und derart mit der Bewegungsvorrichtung verbunden ist, daß der Ankoppelvorgang gestoppt werden kann. Dadurch wird erreicht, daß während des Ankoppelvorganges nicht der Patient bewegt wird, da rechtzeitig beispielsweise der Stoßwellengenerator gestoppt wird. Dadurch kann das Konkrement nicht mehr aus dem Fokusbereich herauswandern.

Die Berührung läßt sich vorteilhaft erkennen, wenn der Detektor einen Ultraschall-Entfernungsmesser aufweist. Einen einfachen Aufbau des Detektors erhält man, wenn er einen durch einen Stößel betätigbaren Schalter aufweist. Eine sichere Erkennung ist bei einer Vorrichtung gewährleistet, bei der zur Regelung bzw. Konstanthaltung des Anpreßdruckes in einem Koppelkörper ein mit Gas füllbarer, elastischer Hohlkörper angeordnet ist, wenn der Detektor einen Drucksensor aufweist, der bei Überschreiten eines vorgebbaren Anpreßdruckes ausgelöst wird.

Die Stoßwellen lassen sich dann auslösen, wenn an dem Detektor ein Rechner angeschlossen ist, der mit der Ortungsvorrichtung und einem Wegaufnehmer für die bewegbaren Komponenten derart verbunden ist, daß er bei Ansprechen des Detektors aufgrund des für die optimale Ankopplung erforderlichen Weges ermittelt, ob das durch die Ortungseinrichtung erkannte Konkrement innerhalb des Wirkungsbereiches des Stoßwellengenerators liegt. Der Untersuchungsperson wird signalisiert, wenn sich das Konkrement nicht mehr im Fokus, sondern im Fokusbereich befindet, wenn der Rechner mit der Markierungsvorrichtung derart verbunden ist, daß eine Einblendung des Wirkungsbereiches erfolgt. Die Untersuchungsperson kann den Ankoppelvorgang auch überprüfen, wenn der Rechner mit der Markierungsvorrichtung verbunden ist und diese derart steuert, daß eine Verschiebung der Zielmarkierung erfolgt. Der Stoßwellengenerator kann in seine stabile Endlage gefahren werden, wenn der Rechner mit Antriebsmitteln der Bewegungsvorrichtung für die Patientenlagerung derart verbunden ist, daß der Patient derart verschoben wird, daß der Ankoppelvorgang fortgesetzt werden kann. Eine einfache manuelle Verschiebung kann erreicht werden, wenn der Rechner mit einer Anzeigevorrichtung verbunden ist, die die Koordinaten der Patientenlagerung für eine optimale Ankopplung anzeigt.

Die Erfindung ist nachfolgend anhand von in der Zeichnung dargestellten Ausführungsbeispielen näher erläutert. Es zeigen:

Fig. 1 einen Lithotripsie-Arbeitsplatz,

Fig. 2 ein Beispiel eines auf dem Monitor dargestellten Röntgenbildes mit Einblendungen nach der Erfindung,

Fig. 3 eine Patientenlagerung mit ausgefahrenem Stoßwellengenerator, und

Fig. 4 einen angekoppelten Stoßwellengenerator gemäß der Erfindung.

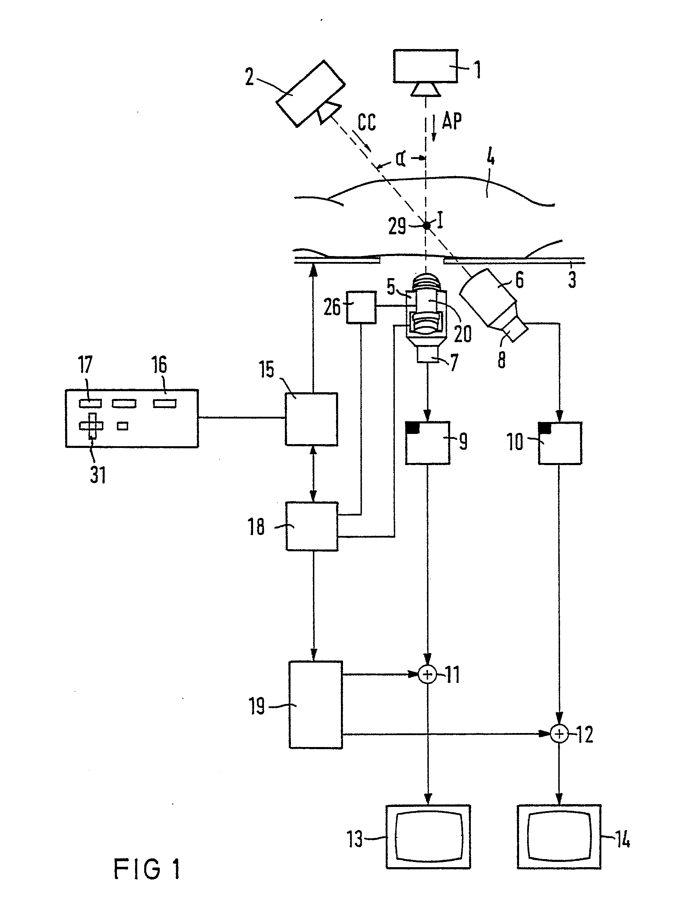

In der Fig. 1 ist ein Lithotripsie-Arbeitsplatz mit einer Rönt gendiagnostikeinrichtung als Ortungsvorrichtung dargestellt, die zwei Röntgenröhren 1 und 2 aufweist, die Röntgenstrahlenbündel erzeugen, die einen auf einer Patientenlagerung 3 befindlichen Patienten 4 durchdringen und auf die Eingangsleuchtschirme von Röntgenbildverstärkern 5 und 6 fallen. Die Röntgenröhre 1 und der Röntgenbildverstärker 5 können dabei derart angeordnet sein, daß der Zentralstrahl des Röntgenstrahlenbündels der Röntgenröhre 1 senkrecht auf den Patienten 4 fällt (a.p.-Projektion). Die Röntgenröhre 2 und der Röntgenbildverstärker 6 sind beispielsweise derart schräg angeordnet, daß der Zentralstrahl der Röntgenröhre 2 den Zentralstrahl der Röntgenröhre 1 im Isozentrum I innerhalb des Patienten 4 unter einem Winkel Alpha von beispielsweise 45° schneidet (c.c.-Projektion). Dadurch erhält man Durchleuchtungsbilder aus zwei verschiedenen Projektionsrichtungen, so daß man den Patienten 4 durch die Patientenlagerung 3 derart dreidimensional verschieben kann, daß sich ein Konkrement 29, beispielsweise ein Nierenstein, in dem Isozentrum I befindet.

Die Ausgangssignale von an den Röntgenbildverstärkern 5 und 6 angekoppelten Fernsehkameras 7 und 8 können in zwei Bildspeichern 9 und 10 eingelesen werden. Die Ausgänge der Bildspeicher 9 und 10 sind mit den ersten Eingängen von Additionsstufen 11 und 12 verbunden, an denen je ein Monitor 13 und 14 zur Wiedergabe der Röntgenbilder angeschlossen ist.

Nach erfolgter Durchleuchtung durch die Röntgenröhren 1 und 2 werden die Röntgenstrahlenbilder von den Bildwandlern, den Bildverstärkern 5 und 6 sowie den angekoppelten Fernsehkameras 7 und 8, in Bildsignale umgewandelt, die in den Bilspeichern 9 und 10 eingespeichert werden. Diesen Bildsignalen wird, wie noch erläutert, in den Additionsstufen 11 und 12 ein Signal zugemischt, so daß auf den Monitoren 13 und 14 die Röntgenstrahlenbilder mit Einblendungen dargestellt werden.

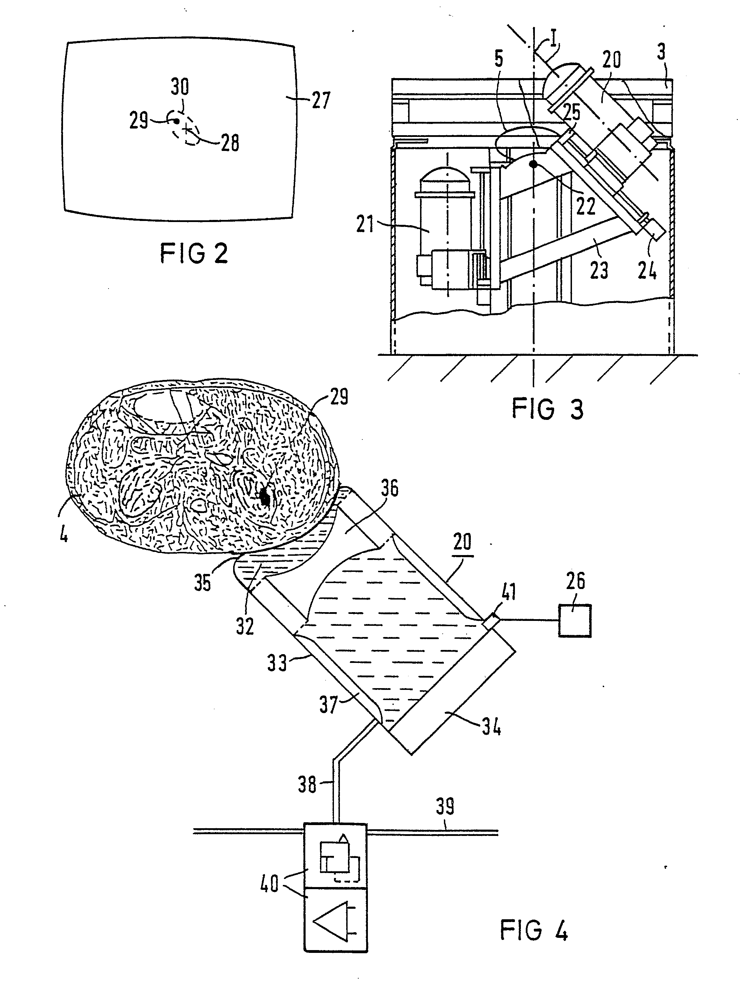

Zur dreidimensionalen Verschiebung der Patientenlagerung 3 ist diese mit einer Steuervorrichtung 15 für deren motorische Ver stellung als Antriebsmittel verbunden. Über ein Bedienpult 16 kann die Patientenlagerung 3 verschoben werden, wobei deren aktuelle Position durch Anzeigemittel 17 für die drei Raumkoordinaten auf dem Bedienpult 16 erkennbar ist. An der Steuervorrichtung 15 ist ein Rechner 18 angeschlossen, der mit einem Signalgenerator 19 als Markierungsvorrichtung verbunden ist, der Einblendungen in ein in Fig. 2 dargestelltes Monitorbild 27 bewirkt. Die Ausgänge des Signalgenerators 19 sind mit den zweiten Eingängen der Additionsstufen 11 und 12 zur Überlagerung mit dem Röntgenstrahlenbild verbunden.

Unterhalb der Patientenlagerung 3 sind Stoßwellengeneratoren 20 und 21 angeordnet, die, wie aus Fig. 3 ersichtlich, nach oben geschwenkt werden und an den Patienten 4 angekoppelt werden können. Die Stoßwellengeneratoren 20 und 21 sind auf einem gemeinsamen, um eine parallel zur Längsachse der Patientenlagerung 3 verlaufende Achse 22 schwenkbaren Halter 23 derart gelagert, daß die Längsachse des eingeschenkten Stoßwellengenerators 20 auf das Isozentrum I ausgerichtet ist. Durch eine Stellvorrichtung 24 als Bewegungsvorrichtung läßt sich der Stoßwellengenerator 20 auf den Patienten 4 zu bewegen. Ist der Stoßwellengenerator 20 an einem Anschlag 25 angekommen, so befindet sich das Isozentrum I im Fokus des Stoßwellengenerators 20.

Ist der Patient 4 aber korpulent, so berührt der feste Teil des Stoßwellengenerators 20 den Patienten, bevor der Stoßwellengenerator 20 den Anschlag 25 berührt. Würde nunmehr der Stoßwellengenerator weiter bis zum Anschlag 25 bewegt werden, so würde der Patient 4 derart verdreht, daß das Konkrement sich nicht mehr im Isozentrum I befindet, so daß eine Stoßwellenbehandlung nicht mehr erfolgen kann. Deshalb ist dem Stoßwellengenerator 20 ein Detektor zugeordnet, der über eine Detektorschaltung 26 mit dem Rechner 18 verbunden ist. Dieser Detektor der Detektorschaltung 26 erkennt die Berührung des Patienten mit dem festen Teil des Stoßwellengenerators 20 und stoppt den Ankoppelvorgang. Der Rechner 18 ist weiterhin entweder mit einer nicht dargestellten Erfassungsschaltung für den Stellwert oder mit der Stellvorrichtung 24 verbunden, die aufgrund ihrer Einschaltdauer dem Rechner 18 ein dem Stellweg entsprechendes Signal zuführt. Daraus ermittelt der Rechner 18 die Lage des Fokus des Stoßwellengenerators 20 und steuert den Signalgenerator 19 beispielsweise derart an, daß die Zielmarkierung, ein Fadenkreuz 28 im Monitorbild 27, verschoben wird. Dadurch liegt das Konkrement 29 nicht mehr im Fadenkreuz 28, so daß die Untersuchungsperson erkennen kann, daß der Ankoppelvorgang vorzeitig gestoppt wurde. Gleichzeitig ermittelt der Rechner 18 den Wirkungsbereich des Stoßwellengenerators 20 und bewirkt über den Signalgenerator 19 eine Einblendung 30 dieses Wirkungsbereiches. Nun kann die Untersuchungsperson feststellen, daß sich das Konkrement 29 noch innerhalb des Wirkungsbereiches 30 befindet, so daß eine Zertrümmerung des Konkrementes 29 durch Auslösen der Stoßwellen erfolgen kann.

In einer anderen Ausführungsform ermittelt der Rechner 18 die erforderlichen Koordinaten für die Patientenlagerung 3, die entweder über weitere Anzeigemittel oder das Monitorbild 27 der Untersuchungsperson angezeigt werden können. Diese Untersuchungsperson kann dann über Steuermittel 31 die Patientenlagerung 3 derart verfahren, daß der Stoßwellengenerator 20 zum vollständigen Ankoppeln bis zum Anschlag 25 bewegt werden kann.

Die Verschiebung der Patientenlagerung kann aber auch automatisch erfolgen, indem der Rechner 18 die Steuervorrichtung 15 entsprechend beeinflußt.

Anhand der Fig. 4 wird nun eine Möglichkeit zur Bestimmung des Berührens des Patienten 3 durch den festen Teil des Stoßwellengenerators 20 beschrieben. In der Fig. 4 ist der Stoßwellengenerator 20 mit einem mit Flüssigkeit 32 gefüllten Stoßwellenrohr 33 dargestellt. Der Stoßwellengenerator 20 weist an seinem unteren Ende eine Stoßwellenquelle 34 auf. Sein Applikationsende ist von einem flexiblen Sack 35 abgeschlossen. Die Applikation erfolgt in der Weise, daß der Stoßwellengenerator 20 mit dem Sack 35 an der Oberfläche des im Querschnitt dargestellten Patienten 4 angelegt wird.

Bei dem dargestellten Beispiel soll ein Konkrement 29, beispielsweise ein Nierenstein, zertrümmert werden. Hierzu werden die von der Stoßwellenquelle 34 erzeugten ebenen Stoßwellen mit Hilfe einer akustischen Linsenanordnung 36 auf den Nierenstein 29 fokussiert, welche gestrichelt eingezeichnete Kanäle aufweist, die den vom Sack 35 umgebenden Raum mit dem Raum vor der Stoßwellenquelle 34 miteinander verbinden.

Zur Einstellung und Regelung des Druckes der Flüssigkeit 32 und damit des Anpreßdruckes am Patienten 4 ist in dem die Flüssigkeit 32 aufnehmenden Raum ein mit Gas, insbesondere mit Luft, füllbarer elastischer Hohlkörper 37 vorgesehen. Der Hohlkörper 37 ist als an der Innenwand des Stoßwellenrohres 33 anliegender, hohler Ring ausgebildet. Der vom Hohlkörper 37 umschlossene Raum ist über eine Leitung 38 mit einer Druckluftleitung 39 und einem Druckregler 40 verbunden.

Mit Hilfe des Druckreglers 40 kann der Druck im Inneren des Hohlkörpers 37 und damit auch der Druck der Flüssigkeit 32 und der Anpreßdruck am Patienten 4 geregelt, insbesondere konstant gehalten werden.

Ist der Patient 4 derart korpulent, daß die Oberfläche seines Körpers auf dem festen Teil des Stoßwellengenerators 20, beispielsweise der Linsenanordnung 36, auftrifft, ist die Luft aus dem Hohlkörper 37 vollständig entwichen, da sich die Anordnung außerhalb ihres Regelbereiches befindet. Wird nun der Stoßwellengenerator 20 weiterhin auf den Körper des Patienten 4 zu bewegt, erhöht sich also der Druck in dem Stoßwellengenerator 20. Dies wird durch einen Drucksensor 41 erfaßt, der mit der Detektorschaltung 26 verbunden ist. Der Rechner 18 stoppt nunmehr den Ankoppelvorgang und bewirkt die weiteren, bereits beschriebenen Maßnahmen.

Anstelle dieses Drucksensors 41 und der Detektorschaltung 26 als Detektor kann aber auch eine Ultraschall-Meßeinrichtung verwendet werden, die mit dem Stoßwellengenerator 20 verbunden ist und die Annäherung des Stoßwellengenerators 20 an den Patienten durch Messung überprüft.

Der Detektor 26 kann aber auch aus einem Schalter bestehen, der durch einen Stößel bewegt wird, bevor der Patient 4 den festen Teil des Stoßwellengenerators 20 berührt.

## Ansprüche

1. Vorrichtung zum berührungslosen Zertrümmern von Konkrementen (29) im Körper eines Patienten (4) mit einem Stoßwellengenerator (20, 21) zur Erzeugung von fokussierten Stoßwellen innerhalb eines in einem Abstand vor dem Stoßwellengenerator (20, 21) liegenden Wirkungsbereiches, wobei der Stoßwellengenerator (20, 21) einen festen Teil (33, 36) und einen mit Koppelflüssigkeit (32) gefüllten Teil (35) aufweist, mit einer Bewegungsvorrichtung (24) zum Ankoppeln des Stoßwellengenerators an einem auf einer Patientenlagerung (3) befindlichen Patienten (4), durch die der Stoßwellengenerator (20, 21) und die Patientenlagerung (3) relativ zueinander bewegt werden, und mit einer Ortungsvorrichtung (5 bis 14) zur Darstellung der Konkremente (29) mit einer Markierungsvorrichtung (19) für die Konkremente, **dadurch gekennzeichnet**, daß der Stoßwellengenerator (20, 21) einen Detektor (26, 41) aufweist, der eine Berührung des Patienten durch den festen Teil (33, 36) des Stoßwellengenerators (20, 21) erkennt und derart mit der Bewegungsvorrichtung (24) verbunden ist, daß der Ankoppelvorgang gestoppt werden kann.

2. Vorrichtung nach Anspruch 1, **dadurch gekennzeichnet**, daß der Detektor (26) einen Ultraschall-Entfernungsmesser aufweist.

3. Vorrichtung nach Anspruch 1, **dadurch gekennzeichnet**, daß der Detektor (26) einen durch einen Stößel betätigbaren Schalter aufweist.

4. Vorrichtung nach Anspruch 1, bei der zur Regelung bzw. Konstanthaltung des Anpreßdruckes in einem Koppelkörper ein mit Gas füllbarer, elastischer Hohlkörper (37) angeordnet ist, **dadurch gekennzeichnet,** daß der Detektor (26, 41) einen Drucksensor (41) aufweist, der bei Überschreiten eines vorgebbaren Anpreßdruckes ausgelöst wird.

5. Vorrichtung nach einem der Ansprüche 1 bis 4, **dadurch gekennzeichnet,** daß an dem Detektor (26) ein Rechner (18) angeschlossen ist, der mit der Ortungsvorrichtung (5 bis 14) und einem Wegaufnehmer (24) für die bewegbaren Komponenten derart verbunden ist, daß er bei Ansprechen des Detektors (26) aufgrund des für die optimale Ankopplung erforderlichen Weges ermittelt, ob das durch die Ortungseinrichtung (5 bis 14) erkannte Konkrement (29) innerhalb des Wirkungsbereiches des Stoßwellengenerators (20, 21) liegt.

6. Vorrichtung nach Anspruch 5, **dadurch gekennzeichnet,** daß der Rechner (1) mit der Markierungsvorrichtung (19) derart verbunden ist, daß eine Einblendung (30) des Wirkungsbereiches erfolgt.

7. Vorrichtung nach Anspruch 5, **dadurch gekennzeichnet,** daß der Rechner (18) mit der Markierungsvorrichtung (19) verbunden ist und diese derart steuert, daß eine Verschiebung der Zielmarkierung (28) erfolgt.

8. Vorrichtung nach Anspruch 5, **dadurch gekennzeichnet,** daß der Rechner (18) mit Antriebsmitteln (15) für die Patientenlagerung (3) derart verbunden ist, daß die Patientenlagerung (3) derart verschoben wird, daß der Ankoppelvorgang fortgesetzt werden kann.

9. Vorrichtung nach Anspruch 5, **dadurch gekennzeichnet,** daß der Rechner (18) mit einer Anzeigevorrichtung (17, 27) verbunden ist, die die Koordinaten der Patientenlagerung (3) für eine optimale Ankopplung anzeigt.

86 P 3468

FIG 1

86 P 3468

FIG 2

FIG 3

FIG 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 169 311 (DORMER)<br>* Zusammenfassung; Seite 8, Zeile 13 - Seite 9, Zeile 40; Seite 10, Zeilen 5-13; Seite 11, Zeilen 3-7; Seite 11, Zeile 21 - Seite 12, Zeile 2; Figur 2 *<br>--- | 1,3-7 | A 61 B 17/22<br>A 61 B 6/10 |
| Y | JP-A-57 151 938 (TOKYO SHIBAURA)<br>* Zusammenfassung *<br>--- | 1,3-7 | |
| Y | DE-A-3 343 924 (SIEMENS AG)<br>* Seite 2, Zeilen 1-33 *<br>--- | 3 | |
| A | DE-A-2 631 102 (SIEMENS AG)<br>--- | | |
| A | EP-A-0 166 102 (DORMER)<br>* Zusammenfassung; Seite 11, Zeilen 7-10 *<br>--- | 1,4 | |
| A | FR-A-2 331 934 (SIEMENS AG)<br>* Seite 2, Zeilen 18-27 *<br>--- | 2 | |
| A | MEDICAL PHYSICS, Band 6, Nr. 4, Juli/August 1979, Seiten 309-311, Am. Assoc. Phys. Med., J.F. HILLS et al.: "Computerized patient contours using the scanning arm of a compound B-scanner"<br>--- | 2 | RECHERCHIERTE SACHGEBIETE (Int. Cl.4)<br><br>A 61 B |
| A | EP-A-0 190 601 (SIEMENS AG)<br>* Spalte 1, Zeilen 20-48; Figur *<br>--- | 4 | |
| A | DE-A-2 925 933 (PICKER)<br>* Figuren *<br>----- | 4 | |

Der vorliegende Recherchenbericht wurde für alle Patentansprüche erstellt

| Recherchenort | Abschlußdatum der Recherche | Prüfer |
|---|---|---|
| DEN HAAG | 11-03-1988 | WOLF C.H.S. |